# EUROPEAN PATENT APPLICATION

(11) **EP 4 706 565 A1**
(43) Date of publication of application: **11.03.2026**
(21) Application number: 25764529.1
(22) Date of filing: 26.02.2025
(51) Int. Cl.: A61B 17/34, A61B 1/00, A61B 1/015, A61B 17/00

(54) **ADAPTER FOR ENDOSCOPIC PORT**

(30) Priority: 10.07.2024 KR 20240091377
(71) Applicant: Dyne Medical Group Inc., Gimpo-si, Gyeonggi-do 10071 (KR)
(72) Inventor: LEE, Sung Hoon, Yeonsu-gu, Incheon 21982 (KR); HWANG, Eun Soon, Gyeonggi-do 10077 (KR)
(74) Representative: Berggren Oy
(86) International application number: PCT/KR2025/002668
(87) International publication number: WO 2026/014646

(57) **Abstract**

According to an embodiment of the present disclosure, a technology is disclosed for an endoscope port adapter that allows adjustment of the position of a surgical instrument received in an endoscope port, and allows simultaneous installation of the surgical instrument and a treatment instrument for supplying saline solution, thereby minimizing the endoscopic procedure time and effectively improving surgical effects.

## Description

### [Technical Field]

The present disclosure relates to an adapter installable in an endoscope port for use in endoscopic procedures, and more particularly, to an endoscope port adapter that adjusts a position of a surgical instrument insertable into an endoscope port.

### [Background Art]

In general, endoscopic surgical instruments refer to instruments used when performing transoral treatments using an endoscope. These endoscopic surgical instruments include a thin and long flexible movable body, a treatment unit configured to perform treatment and provided at an end tip of the movable body insertable into the human body, and an operating unit configured to control the treatment unit and provided at a proximal portion of the movable body that is drawn out to the outside.

Meanwhile, endoscopic surgical instruments using lasers, snares, needles, and knives are configured to be received through the working channel of a flexible endoscope. Here, laser treatment is performed by inserting a thin tube into a target site, inserting an endoscope into the tube, and directly observing a lesion. A snare is used to remove polyps or the like by means of a lasso, a needle is used to inject a predetermined drug into an organic tissue, and a knife is used to cut a predetermined organic tissue such as a lesion.

With the advancement of endoscopy, new treatment methods using endoscope are continuously being developed, and the development of endoscopic instruments suitable for such new treatment methods is required.

In endoscopic resection, various types of instruments such as lasers, injection needles, snares, and knives are used, and these instruments are received through an endoscopic instrument path during a procedure and are frequently replaced with other instruments during the procedure or for subsequent procedures. In particular, the process of inserting a tube for laser treatment into a thin and long endoscopic instrument path, removing the tube, and then inserting another tube requires considerable time and effort.

In addition, since endoscopic surgical instruments are generally received and fixed in the instrument path during a procedure, there have been difficulty in finely adjusting the position of the instruments.

### [Disclosure]

### [Technical Problem]

An object of the present disclosure is to provide an endoscope port adapter that allows fine adjustment of a position of a surgical instrument received into an endoscope port, and allows simultaneous installation of the surgical instrument and a treatment instrument through which saline solution is supplied or suction is performed, thereby minimizing endoscopic procedure time and effectively improving surgical effects.

### [Technical Solution]

According to an embodiment of the present disclosure, an endoscope port adapter detachably mountable to an endoscope port configured to receive a surgical instrument includes: a main pipe configured to receive the surgical instrument and provided with a position adjustment mechanism for the surgical instrument; and a branch pipe branched from the main pipe and configured to inject saline solution, wherein one end of the main pipe is provided with an insertion tube configured to be inserted into the endoscope port and a coupling cap configured to be screw-coupled to the endoscope port; wherein an other end of the main pipe is provided with a hollow movable body configured to allow the surgical instrument to pass therethrough and to reciprocally move by a predetermined distance under control of the position adjustment mechanism, the movable body having a gripper disposed therein and configured to selectively hold the surgical instrument, and a pressing cap configured to be screw-coupled to one end of the movable body and to selectively press the gripper; and wherein the branch pipe is provided with a control valve configured to control a flow rate of the saline solution.

In an embodiment, the movable body may be provided with a seating step to allow the gripper to be seated thereon, and one end of the movable body is provided with a first screw thread configured to be screw-coupled to the pressing cap, the gripper may have a hollow shape to allow the surgical instrument to pass therethrough, may be mounted on the seating step formed inside the movable body, and may be configured to be selectively deformed by pressure by the pressing cap so as to hold the surgical instrument, and the pressing cap may have a hollow shape to allow the surgical instrument to pass therethrough, may be provided with a second screw thread configured to be coupled to the first screw thread formed at one end of the movable body, and may be formed with a pressure projection configured to selectively press the gripper while being screw-coupled to one end of the movable body.

In an embodiment, the position adjustment mechanism may be for finely adjusting a position of a surgical instrument fixed by the gripper, and may include a position adjustment lever having a rack gear portion formed on an outer surface of the movable body and a pinion gear portion rotatably coupled to the main pipe and engaged with the rack gear portion.

In an embodiment, the position adjustment lever may include a pivot shaft portion axially coupled to a coupling opening formed in the main pipe, and a handle portion extending from the pivot shaft portion, the pinion gear portion being formed in the pivot shaft portion.

In an embodiment, the position adjustment lever may be formed with a stopper portion configured to limit a rotation range of the pinion gear portion, and a pair of engaging projections may be formed in the coupling opening of the main pipe so that the stopper portion is caught when the position adjustment lever is rotated.

In an embodiment, the other end of the main pipe may be provided with a flange portion having a wide diameter so as to accommodate the pressing cap during reciprocal movement of the movable body.

In an embodiment, a first O-ring may be mounted between the insertion tube formed at one end of the main pipe and the coupling cap so as to prevent leakage of saline solution, which is supplied through the branch pipe, via the main pipe, and the movable body configured to be inserted into the other end of the main pipe may be provided with a second O-ring.

In an embodiment, in a case where the endoscope port is of a double-pipe type in which a conduit for a surgical instrument and a conduit for saline solution are formed, the branch pipe may be screw-coupled to a closing cap configured to close the saline solution conduit.

According to an embodiment of the present disclosure, an endoscope port adapter detachably mountable to an endoscope port configured to receive a surgical instrument includes: a main pipe configured to receive the surgical instrument and provided with a position adjustment mechanism for the surgical instrument; and a branch pipe branched from the main pipe and configured to receive an other surgical instrument, wherein one end of the main pipe is provided with an insertion tube configured to be inserted into the endoscope port and a coupling cap configured to be screw-coupled to the endoscope port; and wherein an other end of the main pipe is provided with a hollow movable body configured to allow the surgical instrument to pass therethrough and to reciprocally move by a predetermined distance under control of the position adjustment mechanism, one end of the movable body being provided with a first gripper mounting portion to which a first gripper configured to selectively hold the surgical instrument is mounted, and a first pressing cap configured to be screw-coupled to one end of the first gripper mounting portion and to selectively press the first gripper.

In an embodiment, the first gripper mounting portion may have a structure that is screw-coupled to one end of the movable body or a structure that is integrally formed with one end of the movable body.

In an embodiment, in a case where the first gripper mounting structure is screw-coupled to one end of the movable body, one end of the movable body may be provided with a first-first coupling screw thread configured to be screw-coupled to the first gripper mounting portion; the first gripper mounting portion has a hollow shape to allow the surgical instrument to pass therethrough, with the first gripper disposed therein, and one end of the first gripper mounting portion is provided, on an inner surface thereof, with a first-second coupling screw thread configured to be screw-coupled to the first-first coupling screw thread; and the first pressing cap has a hollow shape to allow the surgical instrument to pass therethrough, and is provided with a first-fourth coupling screw thread configured to be screw-coupled to a first-third coupling screw thread formed at an other end of the first gripper mounting portion.

In an embodiment, one end of the branch pipe may be provided with a second gripper mounting portion of a hollow shape configured to allow an other surgical instrument to pass therethrough, the second gripper mounting portion having a second gripper disposed therein and configured to selectively hold the other surgical instrument, and a second pressing cap configured to be screw-coupled to one end of the second gripper mounting portion and to selectively press the second gripper.

In an embodiment, the second gripper mounting portion may be in a structure that is screw-coupled to one end of the branch pipe or a structure that is integrally formed with one end of the branch pipe.

In an embodiment, in a case where the second gripper mounting portion is in a structure that is screw-coupled to one end of the branch pipe; one end of the branch pipe may be provided with a second-first coupling screw thread to be screw-coupled to the second gripper mounting portion, and one end of the second gripper mounting portion may be provided, on an inner surface thereof, with a second-second coupling screw thread configured to be screw-coupled to the second-first coupling screw thread; the second pressing cap may have a hollow shape to allow the other surgical instrument to pass therethrough, an other end of the second gripper mounting portion being provided with a second-fourth coupling screw thread configured to be screw-coupled to a second-third coupling screw thread.

### [Advantageous Effects]

According to the endoscope port adapter of the present disclosure configured as above, the movable body into which the surgical instrument is inserted is inserted and mounted to be reciprocally movable by the position adjustment mechanism provided to the main pipe, so that the movable body can be moved by a distance corresponding to its range of travel. As a result, the position of the surgical instrument received in the movable body may be finely adjusted.

In addition, according to the endoscope port adapter of the present disclosure, the main pipe into which the surgical instrument is insertable and the branch pipe branched from the main pipe are integrally formed, and thus, the surgical instrument and a treatment instrument for supplying saline solution or performing suction may be installed to the endoscope port using a single adapter.

### [Description of Drawings]

FIG. 1 is a view illustrating an example where an endoscope port adapter according to an embodiment of the present disclosure is mounted to an endoscope port into which a surgical instrument is insertable.
FIG. 2 is a perspective view illustrating an endoscope port adapter according to an embodiment of the present disclosure.
FIG. 3 is an exploded perspective view illustrating an endoscope port adapter according to an embodiment of the present disclosure.
FIG. 4 is a cross-sectional view illustrating an endoscope port adapter according to an embodiment of the present disclosure.
FIG. 5 is an exploded cross-sectional view illustrating an endoscope port adapter according to an embodiment of the present disclosure.
FIG. 6 is a cross-sectional view illustrating a movable body and a fixing cap of an endoscope port adapter according to an embodiment of the present disclosure.
FIG. 7 is a perspective view illustrating a main pipe of an endoscope port adapter according to an embodiment of the present disclosure.
FIG. 8 is a perspective view illustrating a position adjustment mechanism according to an embodiment of the present disclosure.
FIG. 9 is a view illustrating a driving process of a pressing cap according to the operation of a position adjustment mechanism of an endoscope port adapter according to an embodiment of the present disclosure.
FIG. 10 is a perspective view illustrating an endoscope port adapter according to another embodiment of the present disclosure.
FIG. 11 is a cross-sectional view illustrating an endoscope port adapter according to another embodiment of the present disclosure.
FIG. 12 is an exploded cross-sectional view illustrating an endoscope port adapter according to another embodiment of the present disclosure.

### [Best Mode for Carrying Out the Invention]

Hereinafter, embodiments disclosed in the present specification will be described in detail with reference to the accompanying drawings and, regardless of the drawing numbers, identical or similar components are assigned the same reference numerals, and redundant descriptions thereof will be omitted for brevity. In addition, in describing the embodiments disclosed in the present specification, a detailed description of well-known related technologies may be omitted if it is deemed to obscure the understanding of the essential features of the embodiments.

Terms such as "first" and "second" may be used to describe various elements but are not to be construed as limiting the elements by such terms. These terms are used solely to distinguish one component from another.

Singular expressions are intended to include plural expressions unless the context clearly indicates otherwise.

Unless otherwise specified by a particular causal relationship, each step described in this application may be performed in an order different from that listed.

As used in this application, terms such as "include" or "have" are intended to specify the presence of stated features, numbers, steps, operations, components, parts, or combinations thereof, but are not intended to exclude the presence or addition of one or more other features, numbers, steps, operations, components, parts, or combinations thereof.

Hereinafter, the present disclosure will be described with reference to the accompanying drawings.

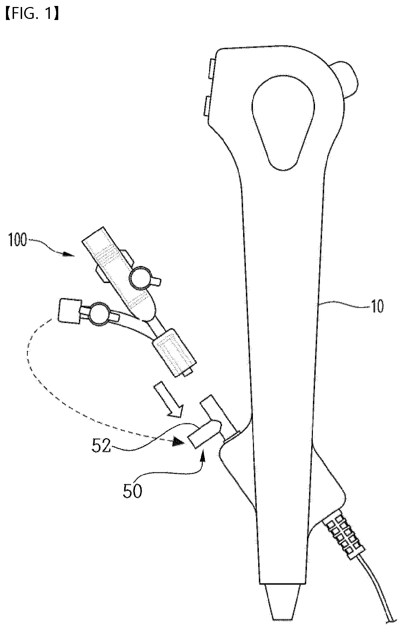

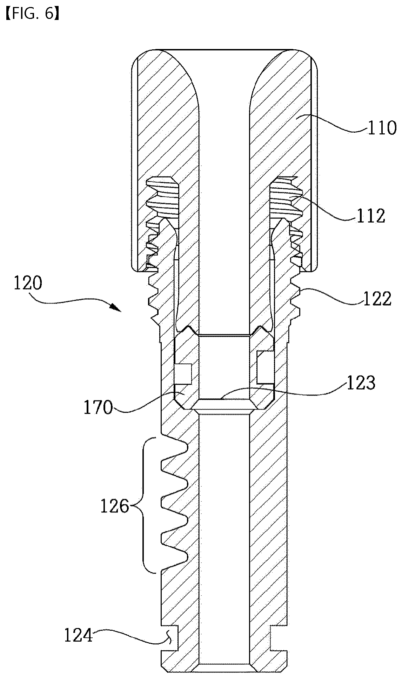

Referring to FIGS. 1 to 9, an endoscope port adapter according to the present disclosure may be configured to adjust the position of a surgical instrument received in the endoscope port, and to allow simultaneous installation of the surgical instrument and a treatment instrument for supplying saline solution, thereby minimizing endoscopic procedure time and effectively improving surgical effects.

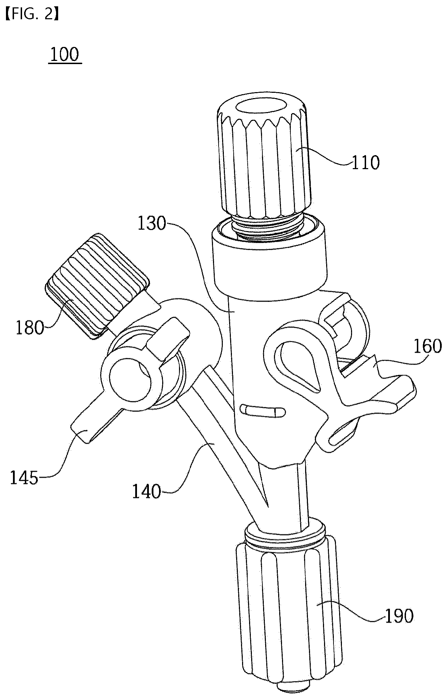

First, an example in which an endoscope port adapter according to the present disclosure is mounted to an endoscope port is described in detail with reference to FIGS. 1 and 2.

An endoscope port adapter 100 according to an embodiment of the present disclosure may be formed to be detachable from an endoscope port 50 into which a surgical instrument is insertable. The endoscope port 50 to which the endoscope port adapter 100 is mounted may be, for example, a device for an esophagogastroduodenoscopy (EGD) procedure, but may alternatively be any one of various devices for other endoscopic procedures.

Here, the endoscope port 50 is described as an example installed in a T-shaped structure, but, the endoscope port 50 may be either straight or T-shaped, and the structure of the endoscope port 50 is not be construed as limiting the present embodiment.

According to an embodiment, the endoscope port adapter 100 may be configured to finely adjust the position of a surgical instrument received in the endoscope port adapter 100. For this purpose, the endoscope port adapter 100 includes a main pipe 130 configured to receive a surgical instrument and provided with a position adjustment mechanism 150 and 160 for the surgical instrument, and a branch pipe 140 branched from the main pipe 130 and configured to inject saline solution.

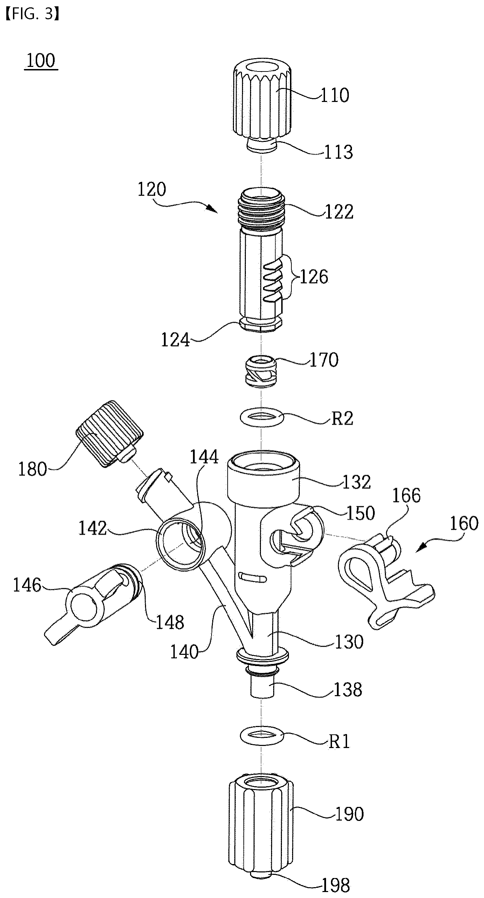

As shown in FIG. 3, one end of the main pipe 130 may be provided with a port insertion tube 198 configured to be inserted into the endoscope port 50, and a coupling cap 190 configured to be screw-coupled to the endoscope port 50. In addition, an other end of the main pipe 130 is provided with a hollow movable body 120 configured to allow the surgical instrument to pass therethrough and to reciprocally move by a predetermined distance under control of the position adjustment mechanism 150 and 160. A gripper 170 for selectively holding a surgical instrument may be disposed in the movable body 120, and a control valve 146 configured to control a flow rate of saline solution may be provided in the branch pipe 140.

Referring to FIGS. 2 to 4 to examine specific features of the main pipe 130 according to the embodiment, the movable body 120 is provided with a seating step 123 to allow the gripper 170 to be seated, and one end of the movable body 120 is screw-coupled to a pressing cap 110. To this end, one end of the movable body 120 may be provided with a first screw thread 122, and the pressing cap 110 may be provided with a second screw thread 112 configured to be coupled to the first screw thread 122 of the movable body 120.

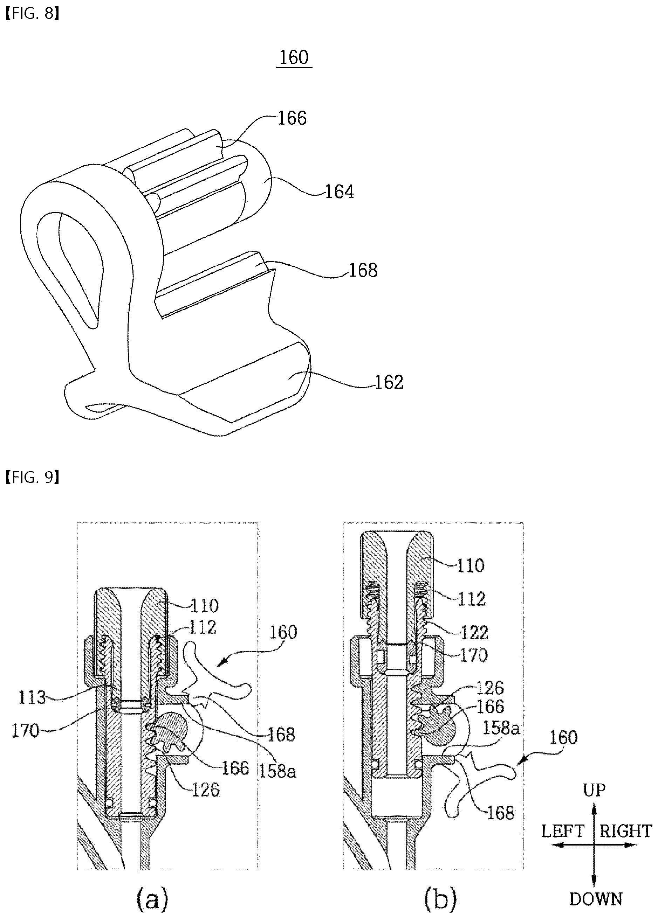

The pressing cap 110 has a hollow shape to allow a surgical instrument to pass therethrough, and is formed with a pressure projection 113 configured to press the gripper 170 while the pressing cap 110 is screw-coupled to one end of the movable body 120. That is, the pressure projection 113 is elongately formed on a lower side of the pressing cap 110 and may be seated on an upper portion of the gripper 170. In the process in which the pressing cap 110 is screw-coupled to one end of the movable body 120, the pressure projection 113 may press the upper portion of the gripper 170, and in this process, the gripper 170 may be selectively pressed (see (a) of FIG. 9).

The gripper 170 has a hollow shape to allow a surgical instrument to pass therethrough, and may be selectively pressed by the pressure projection 113 formed on a lower side of the pressing cap 110 during the process in which the pressing cap 110 is screw-coupled to one end of the movable body 120. In this case, the shape of the gripper 170 may be partially deformed in a direction (e.g., left-right direction) perpendicular to a direction in which the pressure projection 113 presses the gripper 170 (e.g., up-down direction). As the shape of the gripper 170 is partially deformed, the surgical instrument inserted and mounted within the gripper 170 may be fixed in position with respect to the gripper 170.

The seating step 123 is a position at which the gripper 170 is seated within the movable body 120. Specifically, the gripper 170 may be seated on a lower portion of the movable body 120. To this end, based on the lower portion of the movable body 120, an inner diameter of the upper portion of the movable body 120 may be formed to be larger than an inner diameter of the lower portion of the movable body 120. That is, the inner diameter of the lower portion of the movable body 120 is formed smaller than the inner diameter of the upper portion of the movable body 120, so that the gripper 170 is placed on the seating step 123 provided at an upper portion of the lower portion of the movable body 120.

The position adjustment mechanism 150 and 160 provided in the main pipe 130 is configured to finely adjust a position of the surgical instrument fixed to the gripper 170. To this end, the position adjustment mechanism 150 and 160 includes a rack gear portion 126 formed on an outer surface of the movable body 120, and a position adjustment lever 160 rotatably coupled to the main pipe 130 and formed with a pinion gear portion 166 to be engaged with the rack gear portion 126. An engagement distance between the rack gear portion 126 of the movable body 120 and the pinion gear portion 166 of the position adjustment lever 160 may correspond to a reciprocating distance of the movable body 120.

The position adjustment lever 160 may include a pivot shaft portion 164 axially coupled to a coupling opening 150 formed in the main pipe 130, and a handle portion 162 extending from the pivot shaft portion 164, and the pinion gear portion 166 may be formed in the pivot shaft portion 164. That is, when the pivot shaft portion 164 is fitted into the coupling opening 150 and the handle portion 162 rotates, the pinion gear portion 166 and the rack gear portion 126 are engaged with each other to cause the movable body 120 to reciprocally move by a predetermined distance.

### [Mode for Carrying Out the Invention]

According to an embodiment, the movable body 120 may be inserted into the main pipe 130, and the pressing cap 110 may be screw-coupled to one end of the movable body 120 to press the gripper 170, thereby fixing the surgical instrument within the movable body 120. In this case, as shown in (a) and (b) of FIG. 9, when the handle portion 162 is rotated in a direction in which the movable body 120 is inserted (e.g., up-down direction), the pinion gear portion 166 may rotate and then the position of the rack gear portion 126 engaged with the pinion gear portion 166 may be changed. As the position of the rack gear portion 126 engaged with the pinion gear portion 166 changes, the movable body 120 may move in a direction opposite to the direction in which the movable body 120 is inserted.

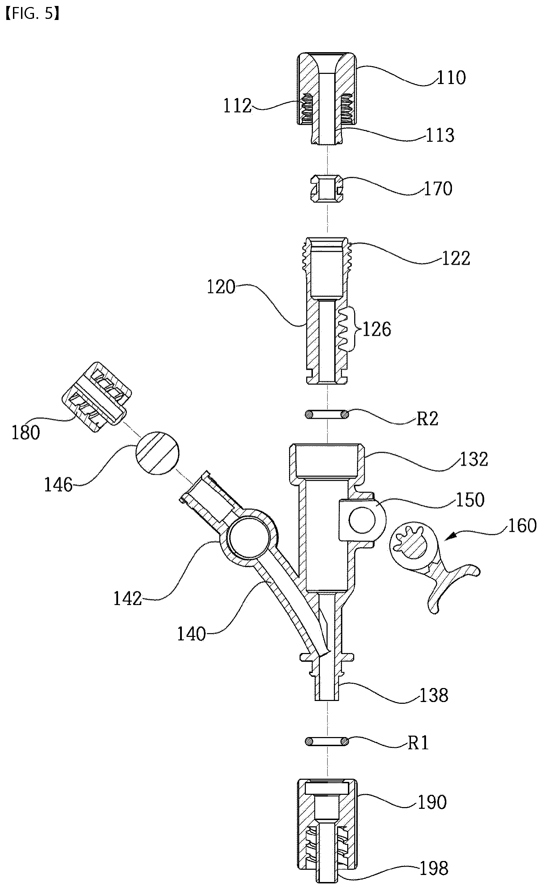

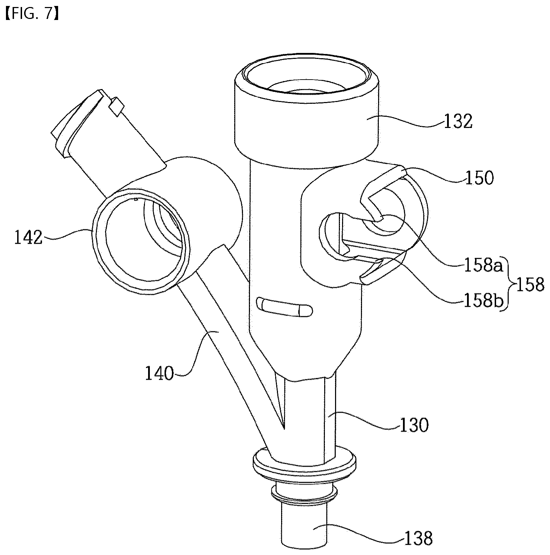

As shown in FIGS. 7 and 8, the position adjustment lever 160 may include a stopper portion 168 configured to limit a rotation range of the pinion gear portion 166. In correspondence with this, as shown in FIG. 5, the coupling opening 150 of the main pipe 130 may be provided with a pair of engaging projections 158a and 158b into which the stopper portion 169 of the position adjustment lever 160 can be caught when the position adjustment lever 160 is rotated.

The stopper portion 169 may limit the range of rotation of the pinion gear portion 166, and when the pinion gear portion 166 is caught by the stopper portion 169 during rotation, the stopper portion 169 may limit continued rotation of the pinion gear portion 166.

In addition, the engaging projection 158 is formed at an end portion of the coupling opening 150, and the pinion gear portion 166 may stop rotating by engaging with the stopper portion 169 during rotation.

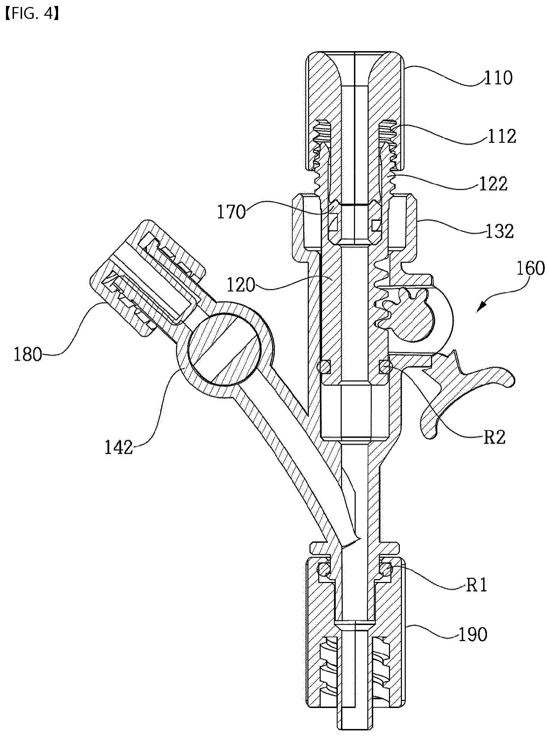

Referring again to FIGS. 4 and 5, an other end of the main pipe 130 may be provided with a flange portion having a wide diameter 132 to accommodate the pressing cap 110 during reciprocal movement of the movable body 120. Specifically, the flange portion 132 may accommodate the pressing cap 110 when the pressing cap 110 is screw-coupled to one end of the movable body 120, thereby preventing the pressing cap 110 from being loosened or damaged due to rotation in a direction opposite to the coupling direction.

Meanwhile, a first O-ring R1 may be provided between an insertion tube 138 formed at one end of the main pipe 130 and the coupling cap 190 to prevent leakage of saline solution, which is supplied through the branch pipe 140, via the main pipe 130, and the movable body 120 configured to be received into the other end of the main pipe 130 may be provided with a second O-ring R2.

Referring to FIG. 5, the insertion tube 138 may be provided with the first O-ring R1 configured to prevent saline solution, which is supplied through the branch pipe 140, from leaking at a joint portion where the insertion tube 138 of the main pipe 130 is mounted.

The second O-ring R2 may prevent saline solution from flowing back between the main pipe 130 and the movable body 120 when the saline solution is supplied through the branch pipe 140. The back flowed saline solution may leak through a gap between the main pipe 130 and the position adjustment mechanism 150 and 160. To prevent such leakage of saline solution, the movable body 120 may be provided with the second O-ring R2 to block the inflow of the saline solution between the main pipe 130 and the position adjustment mechanism 150 and 160. In order to mount the second O-ring R2 on the movable body 120, an other end of the movable body 120 may be provided with an O-ring mounting portion 124.

Referring again to FIGS. 4 and 5, in a case where the endoscope port 50 is of a double-pipe type in which a conduit for a surgical instrument and a conduit for saline solution are formed (see 50 in FIG. 1), the branch pipe 140 is screw-coupled to a closing cap 180 configured to close the saline solution conduit 52.

Specifically, an end of the branch pipe 140 may be coupled to the closing cap 180, and when necessary, the closing cap 180 may be separated; from an end portion of the branch pipe 140 to block the saline solution conduit 52, thereby preventing further supply of saline solution therethrough. In addition, when saline solution is supplied through the branch pipe 140, the saline solution conduit 52 may be closed to prevent external dust from entering the saline solution conduit 52.

The control valve 146 of the branch pipe 140 may selectively open or close the inside of the branch pipe 140, thereby limiting the flow rate of saline solution supplied through the branch pipe. To this end, the control valve 146 may be rotatably installed at the branch pipe 140, and may be rotated to allow communication with the interior of the tubular branch pipe 140 or to block the interior of the branch pipe 140 to limit the flow rate of the supplied saline solution.

A valve installation portion 142 to which the control valve 146 is installed is formed in the branch pipe 140, and the control valve 146 and the valve installation portion 142 may be hook-connected. To this end, one end of the control valve 146 may be provided with a first coupling portion 148, and the valve installation portion 142 may be provided, on an inner surface thereof, a second coupling portion 144 configured to be coupled to the first coupling portion 148.

In this way, when adjusting the position of the surgical instrument using the control valve 146, the flow rate of saline solution supplied to the branch pipe 140 is limited and the inflow and supply of saline solution to the main pipe 130 is blocked.

In one embodiment, the branch pipe 140 is described as limiting the supply of saline solution, and alternatively, a pressure pump may be installed in the branch pipe 140 to automatically initiate suction. While suction is in progress, the control valve 146 is open, and when suction is to be stopped, the control valve 146 may be closed to stop the suction.

According to an embodiment of the present disclosure, in a configuration in which saline solution is supplied through a branch pipe and an other surgical instrument is insertable through the branch pipe, an endoscope port adapter according to another embodiment of the present disclosure will be described in detail with reference to the drawings below.

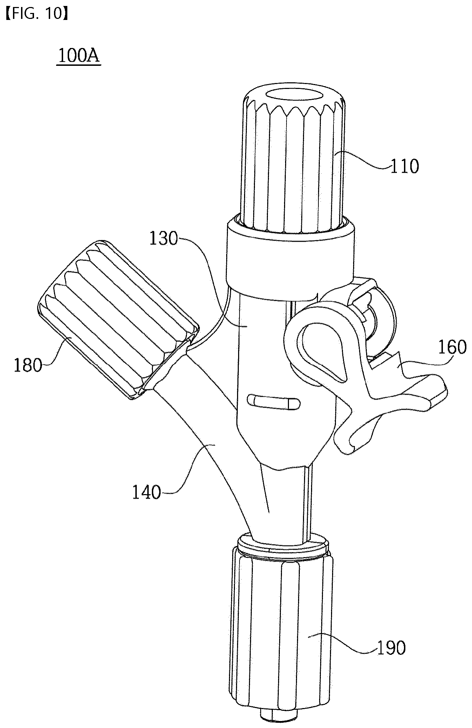

Referring to FIGS. 10 to 12, an endoscope port adapter according to another embodiment of the present disclosure is configured such that, in addition to supplying saline solution through a branch pipe, an other surgical instrument is insertable through the branch pipe, a main pipe into which the surgical instrument is insertable is configured to selectively hold the surgical instrument and to allow fine adjustment of the position of the inserted surgical instrument.

Before providing a detailed description of the drawings, in cases where the configuration of an endoscope port adapter 100A described in FIGS. 10 to 12 according to another embodiment of the present disclosure is the same as an endoscope port adapter 100 described in FIGS. 1 to 9 according to an embodiment of the present disclosure, a detailed description will be omitted.

The endoscope port adapter 100A according to another embodiment of the present disclosure may include a main pipe 130 into which a surgical instrument is insertable, and a branch pipe 140 that branches from the main pipe 130 and into which an other surgical instrument is insertable.

One end of a main pipe 130 of the endoscope port adapter 100A according to another embodiment of the present disclosure may be provided with a port insertion tube 198 configured to be inserted into an endoscope port, and a coupling cap 190 configured to be screw-coupled to the endoscope port.

In addition, an other end of the main pipe 130 may be provided with a hollow movable body 120 configured to allow the surgical instrument to pass therethrough and to reciprocally move by a predetermined distance under control of the position adjustment mechanism 150 and 160.

To this end, one end of the movable body 120 may be provided with a first gripper mounting portion 175A configured to have a first gripper 170A mounted thereto to selectively hold a surgical instrument, and one end of the first gripper mounting portion 175A may be provided with a first pressing cap 110A configured to be screw-coupled thereto so as to selectively press the first gripper 170A.

Specifically, the first gripper mounting portion 175A may be formed either in a structure that is screw-coupled to one end of the movable body 120 or in a structure that is integrally formed with one end of the movable body 120, and in the present disclosure, an example in which the first gripper mounting portion 175A is formed in a screw-coupled structure will be described below.

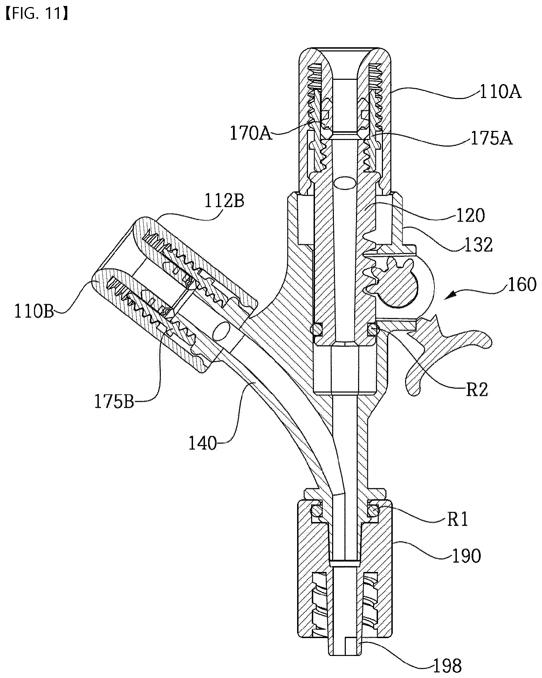

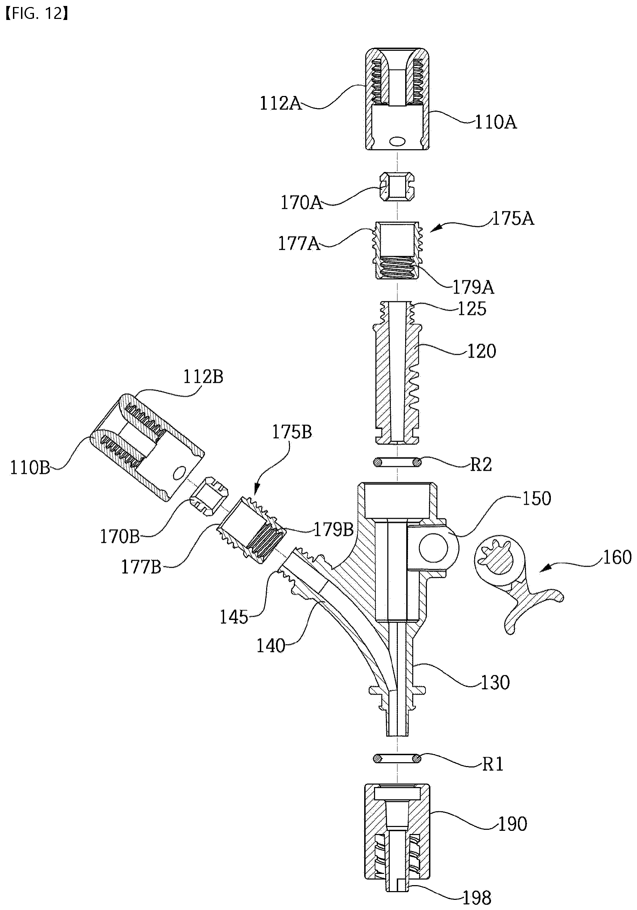

In other words, referring to FIGS. 11 and 12, when the first gripper mounting portion 175A is in a structure that is screw-coupled to one end of the movable body 120, one end of the movable body 120 may be provided with a first-first coupling screw thread 125 configured to be screw-coupled to the first gripper mounting portion 175A, and one end of the first gripper mounting portion 175A may be provided, on an inner surface thereof, with a first-second coupling screw thread 179A configured to be screw-coupled to the first-first coupling screw thread 125. In addition, the first pressing cap 110A may be provided, on an inner surface thereof, with a first-fourth coupling screw thread 112A configured to be screw-coupled to a first-third coupling screw thread 177A formed on the outer surface of an other end of the first gripper mounting portion 175A.

At this time, the first gripper mounting portion 175A may be formed in a hollow shape to allow a surgical instrument to pass therethrough, and the first gripper 170A may be mounted therein. Similarly, the first pressing cap 110A may be formed in a hollow shape to allow a surgical instrument to pass therethrough.

When the first gripper mounting portion 175A is mounted inside the first pressing cap 110A and screw-coupled to the movable body 120, the first gripper 170A may be pressed. To this end, the first pressing cap 110A may include a first gripper pressure portion 113A that is formed inside the first pressing cap 110A and configured to come into contact with an upper portion of the first gripper 170A to press the upper portion of the first gripper 170A mounted in the first gripper mounting portion 175A.

In the process in which the first pressing cap 110A is screw-coupled to the first gripper mounting portion 175A, the first gripper 170A may be selectively pressed by a lower end of the pressing cap 110A that presses the upper portion of the first gripper 170A. The shape of the first gripper 170A may be partially deformed in a direction (e.g., left-right direction) perpendicular to a direction in which the first pressing cap 110A presses the first gripper 170A (e.g., up-down direction). As the shape of the first gripper 170A is deformed, the position of the surgical instrument inserted and mounted in the first gripper 170A is fixed.

Referring again to FIGS. 11 and 12, One end of the branch pipe 140 may have an other surgical instrument inserted and mounted therein, and a gripper may be used to fix the position of the other surgical instrument so that the other surgical instrument can be secured to the branch pipe 140. To this end, the branch pipe 140 may be screw-coupled to a hollow second gripper mounting portion 175B, and a second gripper 170B for selectively holding the other surgical instrument may be mounted in the second gripper mounting portion 175B. At this time, one end of the second gripper mounting portion 175B may have a second pressing cap 110B configured to be screw-coupled thereto to selectively press the second gripper 170B.

As shown, the branch pipe 140 is formed by branching from the main pipe 130, and the other surgical instrument may be inserted and mounted in the branch pipe 140. At this time, in another embodiment, a structure for insertion and mounting of the other surgical instrument may include the second pressing cap 110B, the second gripper mounting portion 175B, and the second gripper 170B.

In another embodiment, the second pressing cap 110B, the second gripper mounting portion 175B, and the second gripper 170B may be formed in either a structure that is integrally formed at one end of the branch pipe 140 or a structure that is screw-coupled to the branch pipe 140 and detachable therefrom, and in another embodiment of the present disclosure, an example in which the structure is screw-coupled will be described.

According to an embodiment, one end of the branch pipe 140 may be provided with a second-first coupling screw thread 145 to be screw-coupled to the second gripper mounting portion 175B, and one end of the second gripper mounting portion 175B may be provided, on an inner surface thereof, with a second-second coupling screw thread 177B configured to be screw-coupled to the second-first coupling screw thread 145.

In addition, the second pressing cap 110B may have a hollow shape to allow an other surgical instrument to pass therethrough, and may include a second-fourth coupling screw thread 112B configured to be screw-coupled to a second-third coupling screw thread 179B formed at an other end of second gripper mounting portion 175B.

That is, according to another embodiment of the present disclosure, the second gripper mounting portion 175B may be mounted by screw-connecting to one end of the branch pipe 140. When the second gripper 170B is mounted in the second gripper mounting portion 175B, the second pressing cap 110B may be screw-coupled to one end of the second gripper mounting portion 175B. As one end of the second gripper mounting portion 175B is screw-coupled to the second pressing cap 110B, the second pressing cap 110B selectively presses an upper end of the second gripper 170B, thereby fixing the position of an other surgical instrument inserted and mounted in the branch pipe 140.

The technical features disclosed in each embodiment of the present disclosure are not limited to that embodiment, and, unless they are mutually incompatible, the technical features disclosed in each embodiment may be combined and applied to different embodiments.

Therefore, although each embodiment focuses on its own technical features, each technical feature may be applied in combination with each other as long as they are not mutually incompatible.

The present disclosure is not limited to the above-described embodiments and the accompanying drawings, and various modifications and variations are possible from the viewpoint of a person skilled in the art to which the present disclosure pertains. Therefore, the scope of the present disclosure should be defined not only by the claims of this specification but also by equivalents thereof.

## Claims

1. An endoscope port adapter detachably mountable to an endoscope port configured to receive a surgical instrument, the adapter comprising:
a main pipe configured to receive the surgical instrument and provided with a position adjustment mechanism for the surgical instrument; and
a branch pipe branched from the main pipe and configured to inject saline solution,
wherein one end of the main pipe is provided with an insertion tube configured to be inserted into the endoscope port and a coupling cap configured to be screw-coupled to the endoscope port;
wherein an other end of the main pipe is provided with a hollow movable body configured to allow the surgical instrument to pass therethrough and to reciprocally move by a predetermined distance under control of the position adjustment mechanism, the movable body having a gripper disposed therein and configured to selectively hold the surgical instrument, and a pressing cap configured to be screw-coupled to one end of the movable body and to selectively press the gripper; and
wherein the branch pipe is provided with a control valve configured to control a flow rate of the saline solution.

2. The endoscope port adapter of claim 1,
wherein the movable body is provided with a seating step to allow the gripper to be seated thereon, and one end of the movable body is provided with a first screw thread configured to be screw-coupled to the pressing cap,
wherein the gripper has a hollow shape to allow the surgical instrument to pass therethrough, is mounted on the seating step formed inside the movable body, and is configured to be selectively deformed by pressure by the pressing cap so as to hold the surgical instrument,
wherein the pressing cap has a hollow shape to allow the surgical instrument to pass therethrough, is provided with a second screw thread configured to be coupled to the first screw thread formed at one end of the movable body, and is formed with a pressure projection configured to selectively press the gripper while being screw-coupled to one end of the movable body.

3. The endoscope port adapter of claim 1, wherein the position adjustment mechanism is for finely adjusting a position of a surgical instrument fixed by the gripper, and comprises a position adjustment lever having a rack gear portion formed on an outer surface of the movable body and a pinion gear portion rotatably coupled to the main pipe and engaged with the rack gear portion.

4. The endoscope port adapter of claim 3, wherein the position adjustment lever comprises a pivot shaft portion axially coupled to a coupling opening formed in the main pipe, and a handle portion extending from the pivot shaft portion, the pinion gear portion being formed in the pivot shaft portion.

5. The endoscope port adapter of claim 4, wherein the position adjustment lever is formed with a stopper portion configured to limit a rotation range of the pinion gear portion, and a pair of engaging projections are formed in the coupling opening of the main pipe so that the stopper portion is caught when the position adjustment lever is rotated.

6. The endoscope port adapter of claim 1, wherein the other end of the main pipe is provided with a flange portion having a wide diameter so as to accommodate the pressing cap during reciprocal movement of the movable body.

7. The endoscope port adapter of claim 1, wherein a first O-ring is mounted between the insertion tube formed at one end of the main pipe and the coupling cap so as to prevent leakage of saline solution, which is supplied through the branch pipe, via the main pipe, and the movable body configured to be inserted into the other end of the main pipe is provided with a second O-ring.

8. The endoscope port adapter of claim 1, wherein, in a case where the endoscope port is of a double-pipe type in which a conduit for a surgical instrument and a conduit for saline solution are formed, the branch pipe is screw-coupled to a closing cap configured to close the saline solution conduit.

9. An endoscope port adapter detachably mountable to an endoscope port configured to receive a surgical instrument, the adapter comprising:
a main pipe configured to receive the surgical instrument and provided with a position adjustment mechanism for the surgical instrument; and a branch pipe branched from the main pipe and configured to receive an other surgical instrument,
wherein one end of the main pipe is provided with an insertion tube configured to be inserted into the endoscope port and a coupling cap configured to be screw-coupled to the endoscope port; and
wherein an other end of the main pipe is provided with a hollow movable body configured to allow the surgical instrument to pass therethrough and to reciprocally move by a predetermined distance under control of the position adjustment mechanism, one end of the movable body being provided with a first gripper mounting portion to which a first gripper configured to selectively hold the surgical instrument is mounted, and a first pressing cap configured to be screw-coupled to one end of the first gripper mounting portion and to selectively press the first gripper.

10. The endoscope port adapter of claim 9, wherein the first gripper mounting portion has a structure that is screw-coupled to one end of the movable body or a structure that is integrally formed with one end of the movable body.

11. The endoscope port adapter of claim 10,
wherein, in a case where the first gripper mounting structure is screw-coupled to one end of the movable body, one end of the movable body is provided with a first-first coupling screw thread configured to be screw-coupled to the first gripper mounting portion;
wherein the first gripper mounting portion has a hollow shape to allow the surgical instrument to pass therethrough, with the first gripper disposed therein, and one end of the first gripper mounting portion is provided, on an inner surface thereof, with a first-second coupling screw thread configured to be screw-coupled to the first-first coupling screw thread; and
wherein the first pressing cap has a hollow shape to allow the surgical instrument to pass therethrough, and is provided with a first-fourth coupling screw thread configured to be screw-coupled to a first-third coupling screw thread formed at an other end of the first gripper mounting portion.

12. The endoscope port adapter of claim 9, wherein one end of the branch pipe is provided with a second gripper mounting portion of a hollow shape configured to allow an other surgical instrument to pass therethrough, the second gripper mounting portion having a second gripper disposed therein and configured to selectively hold the other surgical instrument, and a second pressing cap configured to be screw-coupled to one end of the second gripper mounting portion and to selectively press the second gripper.

13. The endoscope port adapter of claim 12, wherein the second gripper mounting portion is in a structure that is screw-coupled to one end of the branch pipe or a structure that is integrally formed with one end of the branch pipe.

14. The endoscope port adapter of claim 13,
wherein, in a case where the second gripper mounting portion is in a structure that is screw-coupled to one end of the branch pipe;
wherein one end of the branch pipe is provided with a second-first coupling screw thread to be screw-coupled to the second gripper mounting portion, and one end of the second gripper mounting portion is provided, on an inner surface thereof, with a second-second coupling screw thread configured to be screw-coupled to the second-first coupling screw thread; and
wherein the second pressing cap has a hollow shape to allow the other surgical instrument to pass therethrough, an other end of the second gripper mounting portion being provided with a second-fourth coupling screw thread configured to be screw-coupled to a second-third coupling screw thread.
